# EUROPÄISCHE PATENTSCHRIFT

(11) **EP 0 946 229 B1**
(45) Veröffentlichungstag und Bekanntmachung des Hinweises auf die Patenterteilung: **25.07.2001**
(21) Anmeldenummer: 97953871.7
(22) Anmeldetag: 20.12.1997
(51) Int. Cl.: A62D 3/00

(54) **BAKTERIENSTAMM AUREOBACTERIUM sp. K2-17 UND VERFAHREN ZUR MIKROBIELLEN DEKONTAMINATION VON MATERIALIEN, DIE MIT VERBINDUNGEN DER PHENOXYALKANSÄURE-HERBIZID-PRODUKTION BELASTET SIND**
BACTERIAL STRAIN AUREOBACTERIUM. sp K2-17 AND METHOD FOR THE MICROBIAL DECONTAMINATION OF MATERIALS WHICH ARE CONTAMINATED WITH COMPOUNDS FROM PHENOXYALCANOIC ACID-HERBICIDE PRODUCTION
SOUCHE BACTERIENNE AUREOBACTERIUM sp. K2-17 ET PROCEDE DE DECONTAMINATION MICROBIENNE DE MATERIAUX CONTAMINES PAR DES COMPOSES ISSUS DE LA PRODUCTION D'HERBICIDES CONTENANT DE L'ACIDE PHENOXYALCANOIQUE

(30) Priorität: 20.12.1996 DE 19654624
(43) Veröffentlichungstag der Anmeldung: 06.10.1999
(73) Patentinhaber: UFZ-UMWELTFORSCHUNGSZENTRUM LEIPZIG-HALLE GMBH, 04318 Leipzig (DE)
(72) Erfinder: MÜLLER, Roland, D-04347 Leipzig (DE); BABEL, Wolfgang, D-04347 Leipzig (DE)
(74) Vertreter: Ziebig, Marlene, Dr. Dipl.-Chem.
(86) Internationale Anmeldenummer: EP9707162
(87) Internationale Veröffentlichungsnummer: WO9828043

(56) Entgegenhaltungen:
- EP-A- 0 694 611
- DE-C- 4 424 756
- DE-C- 19 608 319
- DE-C- 19 654 624
- CHEMICAL ABSTRACTS, vol. 121, no. 4, 25.Juli 1994 Columbus, Ohio, US; abstract no. 42264, RAMADAN, M. A.: "A variable response of degrading bacteria to phosphorus added to natural water" XP002069059 & J. APPL. BACTERIOL. (1994), 76(4), 314-19 CODEN: JABAA4;ISSN: 0021-8847, 1994,
- DATABASE WPI Section Ch, Week 9709 Derwent Publications Ltd., London, GB; Class D16, AN 97-098614 XP002066825 & RU 2 061 752 C (SARAT IND MICROORGANISMS GENETICS SELECT)
- OH K -H ET AL: "MICROBIOLOGICAL TREATMENT OF FERTILIZER SOLID WASTE MATERIAL CONTAINING PHENOXYALKANOIC HERBICIDES 2,4-D AND MCPP" JOURNAL OF CHEMICAL TECHNOLOGY AND BIOTECHNOLOGY (INTERNATIONAL JOURNAL OF BIOTECHNICAL AND CHEMICAL PROCESSES), Bd. 61, Nr. 4, 1.Dezember 1994, Seiten 299-305, XP000481463
- NAKATSU C ET AL: "GENOMIC REARRANGEMENTS OBSERVED DURING EXPERIMENTAL EVOLUTION OF A 2,4-DICHLOROPHENOXYACETIC ACID DEGRADING COMAMONAS ACIDOVORANS STRAIN TFD41" ABSTRACTS OF THE ANNUAL MEETING OF THE AMERICAN SOCIETY FOR MICROBIOLOGY, 23.Mai 1994, Seite 234 XP000197330
- H. MERTINGK, R.H. MÜLLER, W. BABEL: "Etherolytic cleavage of 4-(2,4-dichlorophenoxy) butyric acid and 4-(4-chloro-2-methylphenoxy) butryric acid by species of Rhodococcus and Aureobacterium isolated from an alkaline environment", JOURNAL OF BASIC MICROBIOLOGY, , -1998, Band 38, Nr. 4, Seiten 257 - 267

## Beschreibung

Die Erfindung betrifft den neuen Bakterienstamm *Aureobacterium sp. K2-17* und ein Verfahren zur mikrobiellen Dekontamination von Materialien, die mit Verbindungen der Phenoxyalkansäure-Herbizid-Produktion belastet sind, wie z.B. mit 2,4-Dichlorphenoxybuttersäure (DCPB), 4-Chlor-2-methylphenoxybuttersäure (MCPB), 2,4-Dichlorphenoxyessigsäure (2,4-D), 4-Chlor-2-methylphenoxyessigsäure (MCPA), 2,4-Dichlorphenol (DCP) und 4-Chlor-2-methylphenol (MCP) in leicht sauren bis stark alkalischen pH-Bereichen.

Produktionsstandorte der chemischen Industrie, in denen jahrzehntelang z.B. Herbizide produziert worden sind und deren Umfeld sind mit den Ausgangs-, Zwischen- und Endprodukten dieser Produktion kontaminiert. Das betrifft sowohl die Böden, Oberflächenwässer und Grundwässer in solchen Arealen, aber auch die Produktionsanlagen und Gebäude selbst. Das gilt u.a. auch für ehemalige Produktionsstandorte der Phenoxyalkansäure-Herbizid-Produktion. Die dadurch vorhandenen Verbindungen, wie z.B. DCPB, MCPB, 2,4-D, MCPA, DCP und MCP sind bekanntermaßen toxisch und haben z.T. cancerogene und teratogene Wirkung. Es führt die Demontage von Chemieanlagen, in denen diese Verbindungen produziert wurden, und die Zerkleinerung des Mauerwerks über Schredderanlagen zu einem Bauschutt, der dekontaminiert werden muß, um diese schädlichen Verbindungen zu beseitigen und um damit gesundheitliche Schäden von exponierten Personen zu vermeiden und auch um Flora und Fauna zu schützen.

Neben kontaminiertem Bauschutt existieren auch mit diesen Substanzen stark belastete wäßrige Abproduktströme. Die Kontaminationen erstrecken sich folglich auch auf Böden und Grundwässer des Umfelds solcher (ehemaligen) Standorte. Deshalb erfordert die Entsorgung von Wässern aus der Produktion solcher Verbindungen besondere Beachtung, da sie die schädigenden Komponenten in konzentrierter Form beinhalten. Es hat sich nämlich gezeigt, daß die Einleitung solcher Produktionswässer in industrielle Kläranlagen zu signifikanten Störungen des biologischen Gleichgewichts und damit der Leistungsfähigkeit dieser Anlagen mit Auswirkungen auf die Geschwindigkeit und den Abbaugrad führen können. Eine effektive Lösung des Problems der Kontaminationen erfordert spezielle Bedingungen und ist an verschiedene Voraussetzungen geknüpft.

Zur Dekontamination fester Matrizes wie Bauschutt sind verschiedene Verfahren auf physikalisch-chemischer Grundlage bekannt. So sind thermische Verfahren zwar effektiv, ihr Nachteil besteht aber darin, daß sie kostenintensiv sind. Desweiteren sind Absaug- oder Waschverfahren mit anschließender absorptiver Schadstoffentfernung, auch im Falle belasteter Wässer, zur Dekontamination fester Matrizes bekannt. Sie verlagern das Problem aber nur auf einen anderen Träger, wenn auch in z.T. hoch konzentrierter Form, und führen somit ebenfalls zu den bereits genannten Nachteilen.

Aufgrund des weitgefächerten metabolischen Potentials von Mikroorganismen werden Dekontaminationsverfahren auf mikrobieller Basis bevorzugt. So sind die mikrobiellen Verfahren in der Regel effektive und kostengünstige Varianten. Es werden dabei die organischen Verbindungen unter Bildung von Biomasse, Wasser, Kohlendioxid und Wärme abgebaut. Dazu können am Standort angesiedelte und an das entsprechende Milieu adaptierte Mikroorganismen genutzt werden. Durch Optimierung der Bedingungen kann das metabolische Potential ausgeschöpft werden. Infolge Vermehrung wird das biokatalytische Potential quasi autokatalytisch sogar gesteigert. Es können aber auch ex situ kultivierte Mikroorganismen (Spezies oder Konsortien) als Starterkulturen kontaminierten Materialien zugesetzt werden, wobei die Sanierungen in unterschiedlichsten Verfahrensregimen durchgeführt werden können (d.h. in situ, on site, off site, Reaktoren, Mieten u.d.g.). Für belastete und konzentrierte Wässer bietet sich eine separate Behandlung und Degradation in Reaktoren an.
Die eigentlichen Standorte, d.h. die Wässer und Grundwässer sowie die Böden können ebenfalls mikrobiell behandelt werden, sei es für im Verlauf der Produktion befindliche Anlagen oder für solche Standorte, die nach dem Einstellen der Produktion und dem Abriß der Anlagen rekultiviert bzw. einer neuen Nutzung zugeführt werden sollen. Nachteilig ist, daß die pH-Werte der Standorte leicht sauer bis alkalisch vorliegen. Wäßrige Eluate aus Betonabbruch sind sogar stark alkalisch, was die Artenvielfalt der Mikroorganismen entsprechend einschränkt und an eine Dekontamination auf mikrobieller Basis besondere Voraussetzungen stellt.

Die produktive Dekontamination von chlorierten und methylierten Phenolen und Phenoxyalkansäuren im neutralen pH-Bereich durch Einzelkulturen (Pieper, D.H. et al. 1988: Arch. Microbiol. 150, 95; Horvath, M. et al. 1990: Appl. Microbiol. Biotechnol. 33, 213; Kilpi, S. 1980: Microbiol. Ecol. 6, 261; Short, K.A. et al. 1990: Can. J. Microbiol. 36, 822; Tiedje, J.M. et al. 1969: J. Agr. Food Chem. 17, 1021) und Konsortien (Bloedorn, I. 1990: Dissertation, Universität Halle; Haugland, R.A. et al. 1990: Appl. Env. Microbiol. 56, 1357; Lappin, H.M. 1985: Appl. Env. Microbiol. 49, 429; Oh, K.H. und Tuovinen, O.H. 1990: J. Ind. Microbiol. 6, 275) ist bekannt. Der Abbau von 2,4-Dichlorphenol sowie 4-Chlor-2-methylphenol durch ein gramnegatives Bakterium, Stamm S1, wurde von Lechner et al. (Biodegradation 6, 1995. 83) beschrieben.

Wie bereits ausgeführt, kommt aber erschwerend hinzu, daß die Kontaminationen in stark alkalischen Medien auftreten. Es ist das Milieu der sog. Alkaliphilen (K. Horikoshi: "Microorganisms in Alkaline Environments", VCH Weinheim, New York 1991). Jüngst konnte gezeigt werden, daß Mikroorganismen, die aus solch kontaminiertem Mauerwerk angereichert worden waren, als Konsortien zum vollständigen Abbau unterschiedlicher Phenoxyalkansäure-Derivate in wäßrigen Eluaten mit pH-Werten bis zu 12,5 befähigt sind (Müller et al., DE 44 24 756.7).

Es sind auch entsprechende alkaliphile Reinkulturen bekannt, die zum Abbau von Phenoxyessigsäure-Derivaten in der Lage sind (Hoffmann et al. 1996: Acta Biotechnol. 16, 121; Maltseva et al. 1996: Microbiology 142, 1115; Müller et al. 1996: ist Internat. Symp. Extremophiles, Estoril, Portugal, p. 208). Monokulturen sind jedoch im Vergleich zu Konsortien in ihrem metabolischen Spektrum eingeschränkt. Sie haben zwar den Vorteil der leichteren Handhabbarkeit, Kontaminationen aus der Herbizid-Produktion beinhalten aber oft ein Spektrum von Phenoxyalkansäure-Derivaten. Mikroorganismen, die auch im Alkalischen in der Lage sind, Phenoxybuttersäure-Herbizide, wie DCPB/MCPB, abzubauen, sind bislang nicht bekannt.

Der Erfindung lag deshalb die Aufgabe zugrunde, ein praktikables und kostengünstiges Verfahren zur mikrobiellen Dekontamination von mit Verbindungen der Phenoxyalkansäure-Herbizid-Produktion belasteten Materialien zu entwickeln und Mikroorganismenstämme zu finden und zu kombinieren, die für den vollständigen Abbau dieser Verbindungen geeignet sind. Insbesondere sollen die Mikroorganismen zur mikrobiellen Dekontamination u.a. von Eluaten aus Abbruchmaterialien von Gebäuden und Anlagen oder von Abwässern und Grundwässern einsetzbar sein.

Es wurde ein neuer Bakterienstamm gefunden, der hervorragend zum Abbau von DCPB und/oder MCPB im leicht sauren bis alkalischen Bereich eingesetzt werden kann. Es handelt sich um den Stamm *Aureobacterium sp. K2-17.* Er wurde am 18. November 1996 bei der Deutschen Sammlung von Mikroorganismen und Zellkulturen GmbH unter der Nummer DSMZ 11288 hinterlegt.

Dieser Stamm wächst im neutralen bis alkalischen Bereich und ist überraschend in der Lage, DCPB und MCPB in die entsprechenden Phenolderivate und C4-Derivate zu spalten.

Die Kultivierung von *Aureobacterium sp. K2-17* erfolgt nach an sich üblichen Methoden kontinuierlich, diskontinuierlich oder mittels anderer Fütterungsstrategien, so z.B. entweder auf einem komplexen Medium, welches Hefeextrakt und/oder Pepton enthält, wobei ein pH-Wert von 7 bis 9,5 vorgelegt werden kann oder unter Nutzung von n-Alkansäuren (Acetat, Butyrat), Dikarbonsäuren (Succinat) oder anderen geeigneten Kohlenstoff/Energie-Quellen. Die Degradation der Phenoxybuttersäuren erfolgt nach Zugabe dieser Verbindungen ohne wesentliche Verzögerung.

Erfindungsgemäß wird in einer bevorzugten Ausführungsvariante dieser neue Stamm *Aureobacterium sp. K2-17* mit einem DCP/MCP mineralisierenden Stamm kombiniert, wodurch der vollständige Abbau der Schadstoffe erreicht wird.

Besonders bevorzugt wird als DCP/MCP verwertender Stamm der Stamm *C. acidovorans* P4a (DSMZ 10474) eingesetzt.

*C. acidovorans P4a* wird ebenfalls nach an sich üblichen Methoden kultiviert, bevorzugt wird er auf 2,4-D bzw. MCPA - unter Hinzufügen von bis zu 0,25 Gewichtsanteilen Hefeextrakt pro Gewichtsanteil Herbizid oder von 1 bis 10 Gewichtsanteilen Na-Acetat oder einer anderen kurzkettigen Alkansäure pro Gewichtsanteil Herbizid - vorzugsweise bei pH 7 bis 9 vermehrt.

Die Kombination des neuen Stammes *Aureobacterium sp. K2-17* mit dem Stamm *C. acidovorans* P4a ermöglicht überraschend den vollständigen Abbau sowohl in leicht sauren und neutralen pH-Bereichen wie auch in stark alkalischem Milieu wodurch z.B. das betroffene Mauerwerk oder entsprechende wäßrige Medien dekontaminiert werden.

Die Degradation der Herbizide durch Wachstum und Vermehrung erfolgt unter aeroben Bedingungen, wobei bevorzugt ein Temperaturregime von 10 bis zu 38 °C gewählt wird. Der pH-Wert liegt vorzugsweise im Bereich von 6-12,5.

Eine weitere Ausführungsvariante besteht in der Kombination von *Aureobacterium sp. K2-17* mit anderen bekannten DCP/MCP-abbauenden Stämmen wie Z. B. dem Stamm S1, der von Lechner et al. in Biodegradation 6, 1995, 83 beschrieben ist, wodurch ein Abbau im leicht sauren bis neutralen pH-Bereich ebenfalls realisierbar ist.

Ein großer Vorteil der Kombination des Stammes *Aureobacterium sp. K2-17* mit *C. acidovorans* P4a besteht zusätzlich darin, daß *C. acidovorans P4a* zum Abbau von 2,4-D und/oder MCPA geeignet ist, wodurch ein breites Spektrum von Verbindungen der Phenoxyalkansäure-Herbizid-Produktion vollständig abgebaut wird.

Die Kultivierung der Stämme wird entweder getrennt oder im Gemisch durchgeführt. In einer bevorzugten Ausführungsvariante erfolgt eine direkte Kultivierung auf DCPB und/oder MCPB, auf gegebenenfalls 2,4-D und/oder MCPA und auf gegebenenfalls DCP und/oder MCP enthaltenden Materialien, wodurch die Verbindungen in Gegenwart an sich üblicher Wachstumskomponenten vollständig abgebaut werden.

Das erfindungsgemäße Dekontaminationsverfahren unter Verwendung von *Aureobacterium sp.* K2-17 kann kontinuierlich erfolgen, indem man mit DCPB und/oder MCPB belastete wäßrige Medien als Kohlenstoff- und Energiequelle anbietet. Wie bereits ausgeführt, ist ein weiterer, DCP/MCP-abbauender, Stamm die Voraussetzung für einen vollständigen Abbau, wobei die an sich üblichen Wachstumskomponenten, wie z.B. Stickstoff und Phosphor, in adäquater Konzentration vorhanden sein müssen.

Mit *Aureobacterium* sp. K2-17 als DCPB und/oder MCPB spaltendem Stamm und *C. acidovorans* P4a als DCP/MCP-Verwerter werden über einem pH-Bereich von 6 bis 10 unter diesen Bedingungen ähnliche stationäre Biomassekonzentrationen für beide Bakterienspezies erhalten. Die Wachstums-, d.h. Substratumsatzraten bewegen sich bei pH-Werten bis zu 8,5 bei ca. 0,05 h⁻¹ bzw. 2,5 mMol/gBTS^{*}h und sinken auf 10-20 % dieser Werte bei pH-Werten um 10 ab. Es erfolgt eine quantitative Degradation dieser Herbizide unter Wandlung in bakterielle Biomasse, Wasser, CO₂, HCl und Wärme.

Die Dekontamination kann auch partiell diskontinuierlich durchgeführt werden, indem man eine entsprechend produzierte Biomasse einem System als Inokulum zugibt, das geeignet ist, die Dekontamination eines Herbizid belasteten Materials in wäßriger Phase zu katalysieren.

Das Verfahren kann im Festbettreaktor, z.B. mittels Mietentechnik oder als Flüssigphasenreaktor betrieben werden, wobei der Prozeß kontinuierlich, partiell kontinuierlich (z.B. fed-batch) oder diskontinuierlich betrieben werden kann. In Abhängigkeit vom gewählten Verfahrensregime können Maßnahmen zur Rückhaltung der Biomasse von Vorteil sein.

Die Biomasse wird in einer Konzentration von vorzugsweise 0,1 bis 1 g/kg einem mit DCPB/MCPB und gegebenenfalls mit 2,4-D und/oder MCPA in einer totalen Konzentration bis zu 2 g/kg kontaminiertem Material in wäßrigem Milieu, welches pH-Werte von ca. 6 bis zu 12,5 aufweisen kann, zugesetzt.

Unter solchen Bedingungen erfolgt die Dekontamination bezüglich der Spaltung von DCPB/MCPB unter intermediärer Bildung von DCP/MCP und deren weiterer vollständiger Mineralisierung mit einer Rate von bis zu 2,5 mMol/gBTS^{*}h, wenn die beiden Spezies zu gleichen Gewichtsanteilen gemischt verwendet werden. Die Rate kann nahezu verdoppelt werden, wenn *Aureobacterium sp. K2-17* und *C. acidovorans* P4a im Verhältnis 2:1 gemischt werden. Diese Umsätze gelten für pH-Werte von ca. 6 bis 8,5. Bei Erhöhung des pH-Wertes bis zu 10 ändert sich das Verhältnis zwischen beiden Spezies nicht wesentlich, die Rate sinkt allerdings auf Werte von 10-20 % ab.

Das erfindungsgemäße Verfahren wird vorteilhaft insbesondere zur Sanierung von Gebäudeabbruch, beispielsweise an Standorten der chemischen Industrie oder mit solchen Substanzen kontaminierten Arealen bzw. Lager- und Umschlagplätzen oder zur Behandlung von Wässern aus der Produktion solcher Verbindungen oder zur Behandlung von mit diesen Herbiziden verunreinigten Böden, Oberflächenwässern und Grundwässern angewendet.

Anschließend wird die Erfindung an Ausführungsbeispielen näher erläutert, ohne sie darauf zu beschränken.

### Beispiel 1

Die Stämme *Aureobacterium sp*. *K2-17* und *Comamonas acidovorans* P4a werden auf einem Minimalmedium angezogen. Dieses Medium hat pro Gramm zu erzeugender Biomasse folgende Zusammensetzung (in mg/l): NH₄Cl, 700; KH₂PO₄ 158; MgSO₄ * 7 H₂O, 8; CaCl₂ * 2 H₂O, 10; FeSO₄ * 7 H₂O, 2,5; ZnSO₄ * 7 H₂O, 0,23; MnSO₄ * 4 H₂O, 0,42; CuSO₄ * 5 H₂O, 0,39; Na₂MoO₄, 0,12.

Die Kultivierung erfolgt im pH-Bereich von 6 bis 10, bei 30° C und einer Gelöstsauerstoffkonzentration von > 30% des Luftsättigungswertes. Als Kohlenstoff- und Energiequelle für das Wachstum von *Aureobacterium sp. K2-17* dienen Hefeextrakt und Pepton in einer Konzentration von je 10 g/l. Die Kultivierung von *C. acidovorans* P4a erfolgt auf 2,4-D und/oder MCPA bzw. deren Salze. Als zusätzliche C/E-Quelle wird Na-Acetat in Gewichtsverhältnis von 1:1-10 angeboten. Es kann auch unmittelbar ein die Komponenten MCPB/DCPB und 2,4-D/MCPA oder/und DCP/MCP enthaltendes Minimalmedium als Kohlenstoff- und Energiequelle zugefüttert werden, wobei beide Stämme gleichzeitig vermehrt werden. Die Wachstumsraten werden auf 0,05 bis 0,1 h⁻¹ eingestellt. Nach Erreichen stabiler Wachstumsbedingungen kann diese Kultur zur Dekontamination von mit Phenoxybuttersäure- und/oder -essigsäure-Derivaten belasteten Wässern eingesetzt werden. Die mit diesen Herbiziden belasteten wäßrigen Medien werden mit Geschwindigkeiten bis zu 0,1 h⁻¹ zugeführt. Der pH-Wert bei stabiler Prozeßführung ist im Bereich von 6 bis 10, vorzugsweise 8 bis 9 zu halten. Sofern die Dekontamination produktiv erfolgt, müssen z.B.

Stickstoff und Phosphor in adäquaten Mengen vorhanden sein. Zuführung von Spurenelementen kann bei Verwendung natürlicher Wässer vernachlässigt werden. Unter diesen Voraussetzungen ist eine komplette Degradation von DCPB/MCPB sowie 2,4-D/MCPA und DCP/MCP bei diesen Geschwindigkeiten möglich. Der Abbau kann über spektrophotomerische oder chromatographische (HPLC) Methoden bzw. über die Freisetzung von z.B. Chlorid oder die Änderung des AOX-Wertes verfolgt werden.

### Beispiel 2

Beide Kulturen werden als Gemisch erzeugt oder in separaten Ansätzen vermehrt. Sie werden in geeigneter Weise auf Trägermatrices aufgebracht, die mit entsprechend Phenoxyalkansäure-haltigen Wässern durchströmt werden. Die Durchflußrate ist einerseits dem biokatalytischen Potential anzupassen, so daß nach Passage ein vollständiger Abbau erfolgt. Andererseits kann der Prozeß durch Rezirkulation bis zur vollständigen Mineralisierung im Kreislauf gefahren werden. Der Prozeß kann als Biomietenverfahren gestaltet werden, wobei nur auf entsprechende Befeuchtung und Belüftung zu achten ist und, wenn sich die Konzentrationen der Phenoxyalkansäuren als hoch im Vergleich zur Inokulumkonzentration erweisen, Makroelementen zuzuführen vorteilhaft ist.

## Patentansprüche

1. Bakterienstamm *Aureobacterium sp. K2-17* (DSMZ 11288).

2. Verfahren zur mikrobiellen Dekontamination von Materialien, die mit Verbindungen der Phenoxyalkansäure-Herbizid-Produktion belastet sind, wie 2,4-Dichlorphenoxybuttersäure (DCPB), 4-Chlor-2-methylphenoxybuttersäure (MCPB), 2,4-Dichlorphenoxyessigsäure (2,4-D), 4-Chlor-2-methylphenoxyessigsäure (MCPA), 2,4-Dichlorphenol (DCP)und 4-Chlor-2-methylphenol (MCP)
dadurch gekennzeichnet, daß
zum Abbau von DCPB und/oder MCPB der Bakterienstamm *Aureobacterium sp. K2-17* (DSMZ 11288) im leicht sauren bis alkalischen Bereich bei pH-Werten zwischen 6 und 12,5 unter aeroben Bedingungen bei Temperaturen zwischen 10 und 38 °C eingesetzt wird.

3. Verfahren nach Anspruch 2,
dadurch gekennzeichnet, daß
*Aureobacterium sp. K2-17* in Kombination mit einem DCP/MCP mineralisierenden Bakterienstamm eingesetzt wird.

4. Verfahren nach Anspruch 3,
dadurch gekennzeichnet, daß
als DCP und/oder MCP mineralisierender Bakterienstamm *Comamonas acidovorans P4a* (DSMZ 10474) eingesetzt wird, der gegebenenfalls auch vorhandene 2,4-D und/oder MCPA abbaut.

5. Verfahren nach Anspruch 4,
dadurch gekennzeichnet, daß
die Stämme *Aureobacterium sp. K2-17* und *Comamonas acidovorans P4a* bei pH-Werten von 6 bis 8,5 im Verhältnis 2:1 eingesetzt werden.

6. Verfahren nach einem der Ansprüche 2 bis 5,
dadurch gekennzeichnet, daß
der Bakterienstamm oder die Bakterienstämme getrennt oder im Gemisch kontinuierlich, diskontinuierlich oder mittels anderer Fütterungsstrategien nach an sich üblichen Methoden kultiviert und anschließend zur Dekontamination in Gegenwart an sich üblicher Wachstumskomponenten eingesetzt werden.

7. Verfahren nach einem der Ansprüche 2 bis 5,
dadurch gekennzeichnet, daß
die Bakterienstämme direkt auf DCPB und/oder MCPB, auf gegebenenfalls 2,4-D und/oder MCPA und auf gegebenenfalls DCP und/oder MCP enthaltenden Materialien, kontinuierlich, diskontinuierlich oder mittels anderer Fütterungsstrategien kultiviert werden, wodurch die Verbindungen in Gegenwart an sich üblicher Wachstumskomponenten abgebaut werden.

8. Verfahren nach einem der Ansprüche 2 bis 7,
dadurch gekennzeichnet, daß
das Dekontaminationsverfahren von einem mit DCPB/MCPB, gegebenenfalls mit 2,4-D/MCPA und gegebenenfalls mit DCP/MCP kontaminierten Material kontinuierlich, partiell kontinuierlich oder diskontinuierlich erfolgt.

9. Verfahren nach einem der Ansprüche 2 bis 8,
dadurch gekennzeichnet, daß
die Dekontamination in einem Festbettreaktor oder einem Flüssigphasenreaktor erfolgt.

## Claims

1. The bacterial strain *Aureobacterium sp. K2-17* (DSMZ 11288).

2. A process for the microbial decontamination of materials polluted with compounds from the production of phenoxyalkanoic acid herbicides, such as 2,4-dichlorophenoxybutyric acid (DCPB), 4-chloro-2-methylphenoxybutyric acid (MCPB), 2,4-dichlorophenoxyacetic acid (2,4-D), 4-chloro-2-methylphenoxyacetic acid (MCPA), 2,4-dichlorophenol (DCP), and 4-chloro-2-methylphenol (MCP), characterized in that the bacterial strain *Aureobacterium sp. K2-17* (DSMZ 11288) in a range of from slightly acidic to alkaline at pH values between 6 and 12.5 under aerobic conditions at temperatures between 10 and 38°C is employed to degrade DCPB and/or MCPB.

3. The process according to claim 2, characterized in that *Aureobacterium sp. K2-17* is used in combination with a DCP/MCP-mineralizing bacterial strain.

4. The process according to claim 3, characterized in that *Comamonas acidovorans P4a* (DSMZ 10474) is employed as DCP- and/or MCP-mineralizing bacterial strain which will also degrade present 2,4-D and/or MCPA, if necessary.

5. The process according to claim 4, characterized in that the strains *Aureobacterium sp. K2-17* and *Comamonas acidovorans P4a* are employed at pH values of from 6 to 8.5 at a ratio of 2:1.

6. The process according to any of claims 2 to 5, characterized in that the bacterial strain or the bacterial strains are cultured separately or in admixture in a continuous or discontinuous fashion, or by means of other feeding strategies according to *per se* usual methods, and subsequently employed for decontamination in the presence of *per se* usual growth components.

7. The process according to any of claims 2 to 5, characterized in that the bacterial strains are directly cultured on materials containing DCPB and/or MCPB, possibly 2,4-D and/or MCPA and possibly DCP and/or MCP in a continuous or discontinuous fashion, or by means of other feeding strategies, so that these compounds will be degraded in the presence of *per se* usual growth components.

8. The process according to any of claims 2 to 7, characterized in that the decontamination process of a material contaminated with DCPB/MCPB, possibly 2,4-D/MCPA and possibly DCP/MCP is conducted in a continuous, partially continuous or discontinuous fashion.

9. The process according to any of claims 2 to 8, characterized in that the decontamination is performed in a fixed-bed reactor or a liquid-phase reactor.

## Revendications

1. Souche bactérienne *Aureobacterium sp. K2-17* (DSMZ 11288).

2. Procédé de décontamination microbienne de matières qui sont chargées avec des composés provenant de la fabrication d'herbicides à l'acides phénoxyalcanoiques ques, comme l'acide 2,4-dichlorophénoxybutyrique (DCPB), l'acide 4-chloro-2-méthylphénoxybutyrique (MCPB), l'acide 2,4-dichlorophénoxyacétique (2,4-D), l'acide 4-chloro-2-méthylphénoxyacétique (MCPA), le 2,4-dichlorophénol (DCP) et le 4-chloro-2-méthylphénol (MCP) caractérisé en ce que ce procédé utilise la souche bactérienne *Aureobacterium sp. K2-17* (DSMZ 11288) pour décomposer le DCPB et/ou le MCPB, en milieu allant de légèrement acide à alcalin, à un pH compris entre 6 et 12,5, dans des conditions d'aérobie, à une température comprise entre 10 et 38° C.

3. Procédé d'après la revendication 2, caractérisé en ce que la souche *Aureobacterium sp. K2-17* est utilisée en l'associant avec une souche bactérienne minéralisante du DCP/MCP.

4. Procédé d'après la revendication 3, caractérisé en ce que la souche bactérienne utilisée en tant que souche minéralisant le DCP/MCP est la souche *Comamonas acidovorans P4a* (DSMZ 10474), qui décompose également le 2,4-D et le MCPA qui sont éventuellement présents.

5. Procédé d'après la revendication 4, caractérisé en ce que les souches *Aureobacterium sp. K2-17* et *Comamonas acidovorans P4a* sont utilisées à un pH de 6 à 8,5 dans un rapport 2 : 1.

6. Procédé d'après une des revendications de 2 à 5, caractérisé en ce que la souche bactérienne ou les souches bactériennes sont élevées dans des milieux de culture suivant des méthodes qui leur sont habituelles, séparées ou en mélange, de manière continue, discontinue, ou par d'autres stratégies de distribution de nourriture et qu'elles sont utilisées ensuite pour la décontamination en présence de milieux de culture qui leur sont habituels.

7. Procédé d'après une des revendications de 2 à 5, caractérisé en ce que les souches bactériennes sont élevées directement sur des matières contenant du DCPB et/ou du MCPB, éventuellement du 2,4-D et/ou du MCPA et éventuellement du DCP et/ou du MCP, de manière continue, discontinue ou au moyen d'autres stratégies de distribution de nourriture, et qu'ensuite les composés sont dégradés en présence de milieux de culture habituels pour ces souches.

8. Procédé d'après une des revendications de 2 à 7, caractérisé en ce que le procédé de décontamination de la matière contaminée par du DCPB/MCPB, le cas échéant par du 2,4-D/MCPA, et éventuellement par du DCP/MCP s'effectue de manière continue, en partie continue ou discontinue.

9. Procédé d'après une des revendications de 2 à 8, caractérisé en ce que la décontamination s'effectue dans un réacteur à lit fixe ou un réacteur à lit fluidisé.
